# EUROPEAN PATENT APPLICATION

(11) **EP 3 494 868 A1**
(43) Date of publication of application: **12.06.2019**
(21) Application number: 17205852.1
(22) Date of filing: 07.12.2017
(51) Int. Cl.: A61B 5/00, A61B 5/04, A61B 5/053, A61B 5/1455

(54) **A SYSTEM FOR SENSING OF A PARAMETER OF A LIVING BEING AND AN ELECTRONIC UNIT FOR USE IN A SYSTEM**

(71) Applicant: IMEC vzw, 3001 Leuven (BE); Stichting IMEC Nederland, 5656 AE Eindhoven (NL)
(72) Inventor: Polito, Salvatore, 3001 Leuven (BE); Myny, Kris, 3001 Leuven (BE)
(74) Representative: AWA Sweden AB

(57) **Abstract**

A system for sensing of a parameter of a living being comprises: a thin film transistor, TFT, unit (110; 310) being configured for attachment to a body part of the living being and said TFT unit (110; 310) comprising at least one sensor (112) with associated sensor circuitry (114) for sensing the parameter of the living being; and an electronic unit (120; 320), wherein the electronic unit (120) and the TFT unit (110) are configured for detachably physically connecting the electronic unit (120) to the TFT unit (110) or arrangement of the electronic unit (320) on the body part in physical contact with the TFT unit (310), wherein the TFT unit (110; 310) and the electronic unit (120; 320) form a wireless contact for powering the TFT unit (110; 310) by the electronic unit (120; 320) and communicating sensing results from the TFT unit (110; 310) to the electronic unit (120; 320).

## Description

### Technical field

The present inventive concept relates to a system for sensing of a parameter of a living being. In particular, the present inventive concept relates to a system comprising a unit carrying at least one sensor and associated sensor circuitry for sensing the parameter, wherein the unit may be attached to a body part.

### Background

To measure biopotentials or other parameters associated to potentials or bioimpedance on the human body, adhesive patches containing electrodes and sensor circuitry are commonly used. These patches are placed at different positions where measurements are to take place. The patches need to be disposable for hygienic reasons and may not be re-used on different persons. Also, electrodes degrade when in contact with skin which may make re-use of patches difficult. For this reason, it is desirable to make an adhesive patch unit as simple as possible in order to minimize costs of the disposable patch unit.

The circuitry in the adhesive patch may be configured to transmit sensor data to a remote unit, such that the adhesive patch may need very limited processing capacity. Further, the circuitry in the adhesive patch may be configured to be powered by the remote unit such that the adhesive patch need not comprise a battery.

In Rose, D.P et al, "Adhesive RFID sensor patch for monitoring of sweat electrolytes", IEEE Transactions on Biomedical Engineering, vol. 62, no. 6, June 2015, pp. 1457-1465, an adhesive radio-frequency identification (RFID) sensor patch is disclosed. The patch is battery-free, powered and read wirelessly by an Android smartphone and custom-app. The adhesive patch includes a Si-based chip. The system of the patch and the smartphone is relatively bulky.

### Summary

It is an object of the present inventive concept to provide a system for sensing a parameter of a living being, wherein the system facilitates continuous monitoring of the living being over a long time frame, while allowing use of inexpensive disposable parts.

These and other objects of the invention are at least partly met by the invention as defined in the independent claims. Preferred embodiments are set out in the dependent claims.

According to a first aspect of the present inventive concept there is provided a system for sensing of a parameter of a living being, said system comprising a thin film transistor (TFT) unit being configured for attachment to a body part of the living being and said TFT unit comprising at least one sensor with associated sensor circuitry for sensing the parameter of the living being, wherein the TFT unit comprises an antenna for short-range communication; and an electronic unit, wherein the electronic unit and the TFT unit are configured for detachably physically connecting the electronic unit to the TFT unit or the electronic unit is configured for fixed arrangement on the body part of the living being in physical contact with the TFT unit, wherein the electronic unit comprises an antenna for short-range communication, wherein the TFT unit and the electronic unit form a wireless contact between the antenna of the TFT unit and the antenna of the electronic unit for powering the TFT unit by the electronic unit and communicating sensing results from the TFT unit to the electronic unit.

Thanks to the system, an electronic unit is provided which may be physically connected to the TFT unit. By physically connecting the electronic unit to the TFT unit, a firm relation between the TFT unit and the electronic unit may be defined which may thus be maintained while the living being goes on with daily life. The electronic unit and the TFT unit may be designed for defining a detachable physical connection between the electronic unit and the TFT unit so as to control the physical relationship between the electronic unit and the TFT unit when physically connected to each other. In this regard, at least one of the electronic unit and the TFT unit may comprise a physical connection interface for defining the detachable physical connection.

The electronic unit may be small and lightweight so as to facilitate the electronic unit being worn by a living being while being physically connected to the TFT unit which is attached to a body part of the living being.

The electronic unit may be configured for fixed arrangement on the body part to which the TFT unit is to be attached. This implies that the system may be designed for arrangement on a body part of the living being such that the TFT unit is attached to the body part and the electronic unit is arranged in a fixed relation to the body part for being worn by the living being. Hence, the system ensures that the TFT unit and the electronic unit, when worn by the living being, are arranged in physical contact, which may ensure that the TFT unit may be powered and sensing results may be received by the electronic unit.

A form factor of the electronic unit may be designed for a specific arrangement of the electronic unit on the living being. For instance, the TFT unit may be formed as a patch to be attached on a wrist or arm of the living being and the electronic unit may be formed as a wristwatch or bracelet for being worn on the arm of the living being. Alternatively, the TFT unit may be formed as a patch to be attached to a chest of the living being and the electronic unit may be formed as a band to be worn over the chest of the living being. According to a further alternative, the TFT unit may be formed as a patch to be attached to a forehead of the living being and the electronic unit may be formed as goggles or glasses to be worn on a head of the living being.

The living being may be a human being. However, the living being may also be an animal, e.g. a pet or a domesticized animal, such as a dog, cat or horse. Thus, the system may be used for monitoring a parameter of a human being or of an animal. In the following, the discussion is mainly done in relation to a human being. However, it should be understood that the system may be similarly used on an animal instead.

Thanks to the TFT unit comprising an antenna and the electronic unit comprising an antenna, the TFT unit and the electronic unit may form a wireless contact through the antennas. The wireless contact may be used for powering of the TFT unit and for communicating sensing results from the TFT unit to the electronic unit.

The electronic unit and the TFT unit may together comprise components which may be useful for allowing gathering of sensing results of a parameter of the living being. By arranging some components in the electronic unit, the TFT unit may be very simple and, hence, inexpensive. For instance, the electronic unit may comprise a battery for powering the TFT unit, which implies that the TFT unit does not need to have a battery. By physically connecting the electronic unit and the TFT unit or placing the electronic unit in physical contact with the TFT unit, it may be ensured that the electronic unit is maintained close to the TFT unit, such that continuous monitoring of the parameter is made possible.

The use of thin film transistor technology of the TFT unit implies that the TFT unit may be inexpensive. The antenna of the TFT unit may be printed on the flexible substrate to provide the possibility of powering the TFT unit and communicating sensing results from the TFT unit. Thus, the antenna may be provided in a simple and inexpensive manner in the TFT unit. Further, the sensor circuitry of the TFT unit may provide elementary functionality in order to be very simple.

In combination, the simple technology provided in thin film transistor technology and the fact that the TFT unit does not need to have any battery implies that the TFT unit may be inexpensive.

Thus, the TFT unit, which should be disposable for hygienic reasons, may be inexpensive. Meanwhile, as the electronic unit is detachably physically connected to the TFT unit, the electronic unit may be detached from the TFT unit after the TFT unit has been used and the electronic unit may then be re-used with another TFT unit. Thus, the electronic unit may be re-usable and it is not as critical that the electronic unit is very inexpensive.

The electronic unit may have a simple configuration so that the electronic unit may be relatively small and lightweight. For instance, even though sensing results are communicated from the TFT unit to the electronic unit, the electronic unit need not include any advanced processing unit or large storage unit for processing and storing the sensing results. Rather, the electronic unit may function as an intermediate unit and further send data to a more advanced external unit. The communication between the electronic unit and the external unit may be performed using a wireless protocol with a relatively large range such that the electronic unit and the external unit need not be immediately close to each other.

Thanks to the wireless contact for powering the TFT unit and communicating sensing results, the contact may be waterproof which allows the system to continue working under wet conditions. Thus, a human being being monitored may not need to be prevented from showering or engaging in other activities involving water during the time of monitoring.

Further, by the use of a thin film transistor unit, the part of the system that is attached to a body part can be made very small, thin and lightweight and can be very flexible so as to form a close fit between the thin film transistor unit and the body part. This also facilitates that the TFT unit can easily be kept attached to a living being in a long time frame.

According to an embodiment, the TFT unit may comprise electrodes, which may form the at least one sensor. The electrodes may thus be configured to acquire a biopotential signal such that a parameter of the living being related to a biopotential measurement may be sensed.

According to an embodiment, the TFT unit may comprise an electrode array comprising a plurality of electrodes. The TFT unit may further comprise a multiplexer and each of the electrodes in the electrode array may be connected to the multiplexer. The TFT unit may thus enable selection of electrodes to be used in acquiring of a biopotential signal by means of selecting output from the multiplexer. This implies that a signal having a desired quality may be selected by selecting which electrodes that are to be used for providing output from the multiplexer. Also, different measurements based on different relations between electrodes in the electrode array may be sequentially acquired by selecting which electrodes to activate by the multiplexer.

However, it is realized that the TFT unit may comprise other types of sensors or additional sensors.

According to an embodiment, the TFT unit may comprise a photo-detector for detecting light incident on the photo-detector. The TFT unit may thus be configured to perform an optical measurement, such as detecting an absorbance or reflectance at the body part to which the TFT unit is attached, such that a parameter of the living being related to the optical measurement may be sensed.

According to one embodiment the TFT unit is formed on a flexible substrate and the sensor circuitry is formed of thin film transistors.

By being formed on a flexible substrate the TFT unit can easily adapt to being attached to a living being. Thin film transistors enable use of material that is very flexible and can be made very thin, while allowing forming of an electric circuitry. Thus, the use of thin film transistors on a flexible substrate allows the TFT unit to be made flexible and therefore suitable for being used in a TFT unit attached to a living being.

The thin film transistors could for instance be formed from an organic material and/or a polymer material. This implies that the TFT unit need not be silicon-based, which may contribute to the TFT unit, which may be disposable, being inexpensive.

According to one embodiment the TFT unit is disposable and the electronic unit is re-usable with different TFT units.

The TFT unit may be inexpensive and, hence, the system may be affordable even though the TFT unit is disposable and intended for one time use only. The TFT unit may be formed from environmentally safe materials only, such that disposing of the TFT unit minimally affects the environment. As the TFT unit is to be used on living beings, the TFT unit being disposable implies that the TFT unit is not allowed to transfer diseases between living beings.

By being re-usable with different TFT units the electronic unit can be used a large number of times, even if the TFT unit is disposed of. Thus, the costs of the system can be kept down. The physical connection between the electronic unit and the TFT unit may be reversible, such that detaching of the electronic unit from the TFT unit enables re-use of the electronic unit.

According to one embodiment the TFT unit is configured to form a waterproof enclosure of the sensor circuitry.

By forming a waterproof enclosure, it may be ensured that the TFT unit can be used in wet conditions. This facilitates using the system for continuous monitoring over a long time as a human being may go on with daily life while wearing the TFT unit. The at least one sensor of the TFT unit, such as electrodes, may protrude from the enclosure so as to allow the at least one sensor to make contact with the body part or receive a signal that is to be measured.

According to one embodiment the TFT unit is a patch, comprising an adhesive for attachment to skin of the living being.

Thus, the TFT unit may be easy to handle as it may be easily attached to a living being. The patch may further be provided with a removable film or any other type of protection for covering the adhesive before use of the patch.

According to one embodiment the sensor circuitry is configured to perform modulation of an analog or digital signal for communicating sensing results to the electronic unit.

This implies that the sensing results may be overlaid as modulations on a carrier signal that may be communicated to the electronic unit. Thus, the communication of sensing results may be performed in a very simple manner, allowing the TFT unit to be based on very simple circuitry.

In some embodiments, the TFT unit may be configured to pre-process sensing results before communicating the sensing results to the electronic unit. Thus, some relatively simple pre-processing may be performed in the TFT unit while still allowing the TFT unit to be overall simple and inexpensive. For instance, the sensor circuitry of the TFT unit may comprise circuitry for converting an acquired analog signal into time domain or into a digital signal and also performing further pre-processing of the acquired signal (e.g. some filtering of the signal).

According to one embodiment the electronic unit comprises a processor unit for processing received sensing results.

By including a processor unit in the electronic unit the sensing results may be processed by the electronic unit. This implies that the electronic unit may analyze the sensing results, e.g. to extract features relating to the parameter of the living being. Thus, the electronic unit may store and/or forward only processed sensing results, which may imply that a smaller amount of data may need to be stored and/or transmitted.

The processor unit may be configured to perform basic analysis of the sensing results, e.g. in order to provide sensing results in a manner that may be easily understood or reviewed by a doctor or a user of the system. However, the processor unit may not necessarily be configured to perform deeper analysis of the sensing results. Rather, advanced analysis which may require relatively large processing resources may be performed in an external unit that may eventually receive the sensing results.

According to one embodiment the electronic unit comprises a memory for storing sensing results.

This allows the retrieval and further processing of the sensing results. The sensing results may be stored as such in the memory, e.g. as raw data. Alternatively or additionally, sensing results that have been processed by the processing unit may be stored in the memory.

The memory may function as a buffer memory in order to temporarily store sensing results that are to be transmitted to an external unit. Thus, the electronic unit may be set up to simply forward any sensing results to an external unit, e.g. through wireless communication with the external unit. The memory may then be used as a buffer memory for storing recent sensing results. Thus, it may be ensured that sensing results are not lost if a connection to the external unit is temporarily lost or data transfer is too slow.

Alternatively or additionally, the memory may function as a relative long-term memory, wherein all sensing results acquired during a session of use of the system may be stored in the memory. Thus, the electronic unit may not necessarily need to communicate with an external unit during the session of acquiring sensing results.

According to one embodiment the electronic unit comprises a communication unit for communication with an external unit.

This allows the transfer of sensing data and/or results to an external unit which can have increased storage and/or processing power compared to the electronic unit, allowing further processing of the sensing results and/or data. The external unit may be remote from the electronic unit and may thus not even be worn by the living being that is monitored.

When the electronic unit comprises a communication unit, the electronic unit may not even need a processing unit. The electronic unit having a communication unit may be set up to continuously transfer sensing results through the communication unit to an external unit, such that no processing of sensing results is necessary in the electronic unit.

However, in another embodiment, the communication unit need not necessarily be configured for wireless communication to the external unit. On the contrary, the communication unit may comprise an output port for physically connecting the external unit to the electronic unit. Thus, the sensing results may for instance be communicated to the external unit after a session of acquiring sensing results, e.g. by plugging a universal serial bus (USB) stick in the output port. The electronic unit may advantageously be provided with a cover or cap for protecting the output port and the electronic unit during acquiring of sensing results, e.g. so as to prevent water from entering the electronic unit.

According to one embodiment the electronic unit comprises a battery.

This allows continuous powering of the system. The battery allows storing of energy in the electronic unit, such that the electronic unit may provide power to the system during an entire session of acquiring sensing results, such as for an entire day or even longer.

According to one embodiment the battery is rechargeable and the electronic unit is configured for wireless charging of the battery.

This allows the charging of the battery without any physical connections. Thus, the electronic unit need not comprise a connection for plugging an external power source to the electronic unit, and there may therefore not be a risk that the electronic unit is damaged by water entering the connection during use.

According to another embodiment, the electronic unit is configured for charging of the battery through a physical connection to a power source. An output port in which the physical connection is formed may be covered during acquiring of sensing results, so as to protect the electronic unit. For instance, the battery may be charged while connecting an external unit to the electronic unit for transferring sensing results to the external unit. Thus, the battery may be charged by a USB stick.

According to one embodiment the electronic unit comprises a housing providing a waterproof enclosure of components of the electronic unit.

This allows the electronic unit to continue to function in wet environments. The waterproof enclosure may completely seal the electronic unit and protect the components of the electronic unit from outer conditions. Since the electronic unit may be configured to power the TFT unit and receive the sensing results through a wireless contact and the electronic unit may further be configured for wireless charging of the battery and wireless communication with an external unit, the waterproof enclosure may be provided without any openings or connections through the housing, while still allowing the electronic unit to have a desired functionality.

According to one embodiment the wireless contact between the TFT unit and the electronic unit is formed through an inductive or capacitive coupling between the antenna of the TFT unit and the antenna of the electronic unit.

An inductive or capacitive coupling allows the TFT unit and the electronic unit to form a wireless connection for transferring power to the TFT unit and sensing results to the electronic unit. This allows the TFT unit and electronic unit to be in contact without any openings of the TFT unit or electronic unit. The units can thus be made waterproof and more robust while still allowing them to be in contact.

Further, having a wireless contact based on inductive or capacitive coupling implies that the antennas in the TFT unit and the electronic unit may be very simple.

According to one embodiment communication between the TFT unit and the electronic unit is performed over the radio frequency identification (RFID) communication or near field communication (NFC).

RFID communication or NFC are suitable protocols for providing a wireless, short-range connection between the TFT unit and the electronic unit. Thus, these types of communication are well suited for use by the TFT unit and the electronic unit, which are configured to be in close relation to each other.

According to one embodiment the electronic unit and the TFT unit are configured for detachably physically connecting the electronic unit to the TFT unit by using at least one: an adhesive, a magnetic coupling, and one or more interlocking elements, such as hook and loop fasteners.

A user may thus easily apply the electronic unit into connection with the TFT unit using connecting means which are easy to handle. The physical connection may provide a firm relation between the TFT unit and the electronic unit such that the connection may be maintained even when the living being wearing the TFT unit is moving around. These types of detachable physical connections are suitable alternatives for providing a physical connection that is easily created and also easily detached without damaging the TFT unit and/or the electronic unit.

According to a second aspect of the present inventive concept there is provided an electronic unit for use in a system for sensing of a parameter of a living being, said electronic unit comprising: a physical connection interface configured for detachably physically connecting the electronic unit to a thin film transistor (TFT) unit; and a wireless communication unit for forming an inductive or capacitive coupling to the TFT unit for powering the TFT unit and receiving sensing results from the TFT unit.

Effects and features of this second aspect are largely analogous to those described above in connection with the first aspect. Embodiments mentioned in relation to the first aspect are largely compatible with the second aspect.

The electronic unit may ensure that a simple TFT unit may be used. The electronic unit may be re-usable and may therefore ensure that a disposable part of a system may be maintained as inexpensive as possible.

The electronic unit may function as an intermediate unit between the TFT unit and an external unit having extensive processing resources. Thus, the electronic unit may also be relatively simple and only provide limited functionalities so as to ensure that the electronic unit is small and lightweight allowing the electronic unit to be physically connected to the TFT unit while the TFT unit is used for sensing a parameter of a living being.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a system according to a first embodiment.
Fig. 2 is a schematic view illustrating a physical connection of a thin film transistor unit and an electronic unit of the system of the first embodiment.
Fig. 3 is a schematic view of internal components of the thin film transistor unit and the electronic unit.
Fig. 4 is a schematic view of a system according to a second embodiment.

### Detailed description

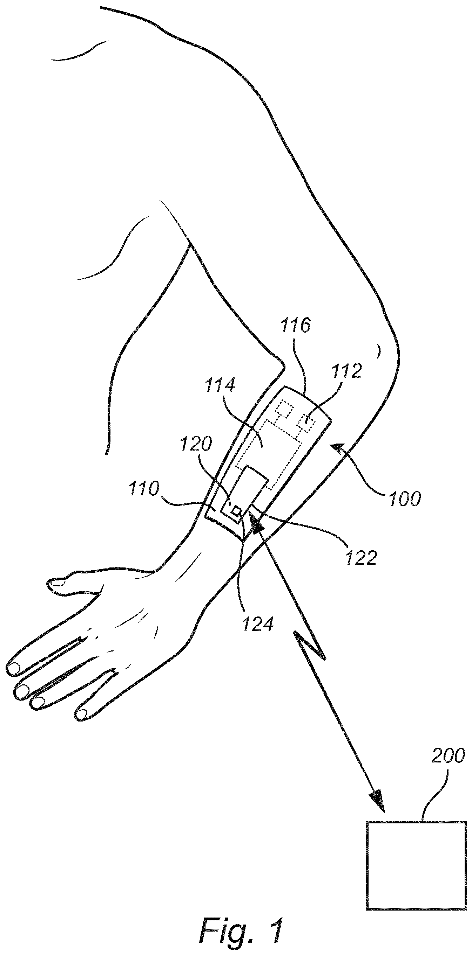

Fig. 1 illustrates a system 100 according to a first embodiment for sensing a parameter of a living being. The system 100 includes a thin film transistor (TFT) unit 110 and an electronic unit 120. An external unit 200 is also shown in Fig. 1. The external unit 200 may be part of the system 100 and may, hence, be a specially adapted unit for co-operating with the other parts of the system 100. However, the external unit 200 need not necessarily be part of the system 100 and may be any conventional external unit 200 having data processing capability, possibly provided with a specific software application relating to the system 100 for sensing a parameter of a living being.

The TFT unit 110 is configured for being attached to a living being, e.g. a human being. The electronic unit 120 and the TFT unit 110 are detachably physically connected to each other. The TFT unit 110 and the electronic unit 120 are together configured to monitor a living being and gather sensing results of a parameter of the living being. Some of the components for monitoring and gathering may be situated in the TFT unit 110 and some may be situated in the electronic unit 120. In order to keep down costs of a TFT unit 110, as few components as possible may be situated in the TFT unit 110.

The system 100 may be designed such that the TFT unit 110 is disposable and the electronic unit 120 is re-usable. When a TFT unit 110 is disposed of, the TFT unit 110 and the electronic unit 120 are detached from each other and the electronic unit 120 can be detachably physically connected to a new TFT unit 110. The new TFT unit 110 can be attached to the same human being, as the TFT unit 110 which was disposed was attached to, or to another human being. Thus, the electronic unit 120 may be re-used for different sessions of sensing a parameter of a human being, either for the same person or for different persons.

The electronic unit 120 can be connected to an external unit 200, either by a wire or wirelessly. The electronic unit 120 may thus transfer sensing results to the external unit 200, which may have larger processing resources for processing and/or analyzing the sensing results. Thus, the electronic unit 120 may be relatively simple and need not comprise capacity to perform complex processing of sensing results.

Transfer of sensing results to the external unit 200 may be performed in real-time. In such an embodiment, the electronic unit 110 may advantageously be connected to the external unit 200 using a wireless connection. This implies that the external unit 200 need not be connected through a physical wire to the electronic unit 120 during gathering of sensing results.

Alternatively, transfer of sensing results to the external unit 200 may be performed after a session of gathering sensing results has been completed. The electronic unit 120 may then be detached from the TFT unit 110, before being connected, through a wired or wireless connection, to the external unit 120 for transferring sensing results.

The TFT unit 110 includes at least one sensor 112 and associated sensor circuitry 114 for sensing a parameter of a human being. According to an embodiment, the at least one sensor 112 may comprise electrodes adapted for sensing a biopotential or bioimpedance signal of the human being. According to another embodiment, the at least one sensor 112 may comprise a photo-detector for detecting incident light on the photo-detector. The photo-detector may thus be configured to make an optical measurement, e.g. for determining a reflectance or absorbance of a body part, such as tissue, of the human being to which the TFT unit 110 is attached.

The TFT unit 110 and the electronic unit 120 form a wireless contact when the electronic unit 120 is physically connected to the TFT unit 110. The electronic unit 120 powers the TFT unit 110 through this wireless contact. Sensing results are also communicated from the TFT unit 110 to the electronic unit 120 through this wireless contact. As mentioned above, the electronic unit 120 may communicate sensing results to the external unit 200.

Thanks to the wireless contact being formed between the TFT unit 110 and the electronic unit 120, the TFT unit 110 may comprise a watertight housing 116 enclosing and protecting the sensor circuitry 114 while allowing the TFT unit 110 to be powered and to communicate with the electronic unit 120. Likewise, the electronic unit 120 may also comprise a watertight housing 122 enclosing and protecting components of the electronic unit 120. This implies that both the TFT unit 110 and the electronic unit 120 may be used in wet environments, such that the TFT unit 110 and the electronic unit 120 may e.g. be worn by a human being while showering.

The electronic unit 120 may further comprise a battery 124. The electronic unit 120 may thus be configured to power the TFT unit 110 by means of energy stored in the battery 124. In order for the electronic unit 120 to be re-usable a number of times, the battery 124 may be rechargeable. The electronic unit 120 may be configured for allowing the battery 124 to be wirelessly recharged. This implies that the electronic unit 120 need not comprise a battery lid for allowing changing batteries or a port for allowing the electronic unit 120 to be plugged to an external power source for charging the battery 124. Hence, the battery 124 may be arranged inside a watertight housing, which facilitates use of the electronic unit 120 in a wet environment.

The system 100 may enable continuous monitoring of a parameter of a human being using an inexpensive disposable TFT unit 110 and a re-usable electronic unit 120, which provides further functionalities that are not provided by the TFT unit 110.

The TFT unit 110 and the electronic unit 120 are configured to be worn by a human being such that the TFT unit 110 and the electronic unit 120 will not affect the human being in daily life while the sensing results are gathered. Hence, no wired connection to an external unit 200 is needed during gathering of sensing results or an external unit 200 need not be in close proximity to the TFT unit 110 or the electronic unit 120 during gathering of sensing results.

Further, the TFT unit 110 and the electronic unit 120 may comprise watertight enclosures such that the TFT unit 110 and/or the electronic unit 120 will not be damaged even if they come in contact with water, such as if the human being is taking a shower while wearing the TFT unit 110 and the electronic unit 120.

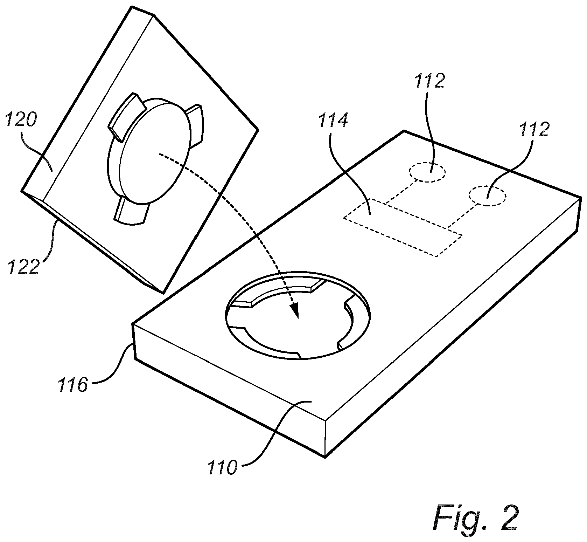

Fig. 2 illustrates a TFT unit 110 and an electronic unit 120. The TFT unit 110 and the electronic unit 120 may be detachably physically connectable to each other.

The TFT unit 110 may be made small, thin and lightweight, such that it easily may be attached to and carried by a human being while the human being goes on with daily life.

The TFT unit 110 may have the form of a patch. The TFT unit 110 may have a first side and a second side. The first side may be configured to be attached to a human being. If so, it may be attached such that the attachment can withstand water, moisture and/or damp environments. The first side may be attachable by an adhesive. The TFT unit 110 may be suitable to be attached to a human being. The first side may be attachable to a human being such that it can be detached and reattached.

The TFT unit 110 may comprise a flexible substrate. The flexible substrate may be an organic material. The flexible substrate may alternatively be a polymer material. The substrate may be flexible in a sense such that the substrate may easily adapt to and take a shape so as to follow a contour of a human being, such as a portion of skin of the human being, e.g. on an arm of the human being. The substrate may further be flexible in that the substrate may change shape so as to adapt to changes of the shape of the portion of skin while the human being moves around and goes on with daily life.

The TFT unit 110 may be waterproof. The TFT unit 110 may provide a watertight enclosure of components within the TFT unit 110 in several manners. For instance, the flexible substrate may provide a water barrier to components, such as the sensor circuitry 114 which may be printed on the flexible substrate. Another layer of flexible water repellent material may be added on top of the components such that components within the TFT unit 110 are surrounded by watertight material. In another embodiment, a separate outer housing 116 of the TFT unit 110 may be tight so that water or moisture is not allowed to pass through the outer housing 116. For instance, the outer housing 116 may be a single part and thus have no joints between parts.

The at least one sensor 112 of the TFT unit 110 may extend through the casing. For instance, if the at least one sensor 112 comprises electrodes, the electrodes may extend through the casing so as to allow the electrodes 112 to be in direct contact with the human being, e.g. with skin of the human being.

If the at least one sensor 112 need not be in direct contact with the human being, the at least one sensor 112 may be arranged within the casing. For instance, if the casing is transparent, a photo-detector may be arranged inside the casing while enabling the photo-detector to receive light.

The second side of the TFT unit 110 may be configured such that the TFT unit 110 may form a detachable physical connection with an electronic unit 120. For instance, the TFT unit 110 may comprise a physical connector interface for providing an interface for connecting to the electronic unit 120. The physical connector interface may include an element on an outer surface, wherein the element is configured for interacting with a corresponding element on the electronic unit 120. Similarly, the electronic unit 120 may comprise a physical connector interface for providing an interface for connecting to the TFT unit 110.

The TFT unit 110 and the electronic unit 120 may be detachably physically connected to each other by an adhesive, a magnetic coupling, a hook and loop fastening or one or more interlocking elements. The detachable physical connection may be achieved based on an element which is arranged on just one of the two units. Alternatively, the detachable physical connection may be achieved based on cooperating elements which are arranged on each of the units. The detachable physical connection may for instance be in the form of a snap fastener, as schematically illustrated in Fig. 2. As an alternative, the TFT unit 110 and the electronic unit 120 may comprise a threaded part such that the detachable physical connection may be formed by screwing the electronic unit 120 into or onto the TFT unit 110. As another alternative, the detachable physical connection may be formed between a protrusion formed on one of the TFT unit 110 and electronic unit 120 and a matching indentation formed on the other one of the TFT unit 110 and electronic unit 120. As yet another alternative, the detachable physical connection may be formed by a clip connection, the clip situated on one of the TFT unit 110 and the electronic unit 120. The detachable physical connection may be strong and robust enough that the TFT unit 110 and the electronic unit 120 remains connected while the system is attached to a human being.

The electronic unit 120 may be made small and lightweight such that it easily may be carried by a human. Thus, the electronic unit 120, when physically connected to a TFT unit 110 that is attached to the human being, does not obstruct the human being in daily life.

The electronic unit 120 may be waterproof, such that the electronic unit 120 may comprise outer layers of water repellent material enclosing components of the electronic unit 120. For instance, the electronic unit 120 may comprise an outer housing 122 being tight so that water or moisture is not allowed to pass through the outer housing 122. For instance, the outer housing 122 may be a single part and thus have no joints between parts. All components of the electronic unit 120 may be arranged inside the housing 122 so as to ensure that the housing 122 is waterproof. It should be realized that some components may partly extend through the housing 122, e.g. a light guide for providing a user interface, such as allowing a status of the electronic unit 120 to be indicated.

The detachable physical connection may also be waterproof, meaning that the detachable physical connection itself then should not be affected by water, moisture or damp environments. However, as there is no need of electrical components in the physical connection between the TFT unit 110 and the electronic unit 120, the detachable physical connection need not be waterproof.

Both the TFT unit 110 and the electronic unit 120 may be robust enough that they withstand the wear and tear that comes with being attached to a human being while the human being goes on with daily life. As the electronic unit 120 may be re-used, the electronic unit 120 may be especially robust to withstand the wear and tear from long-time use while being attached to a human being.

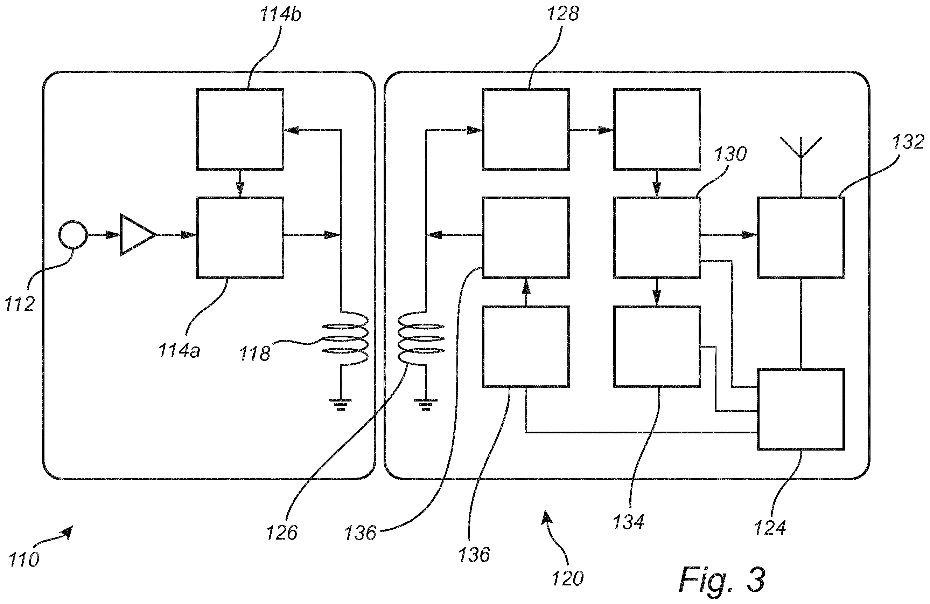

Fig. 3 illustrates a TFT unit 110 and an electronic unit 120 with their respective components in relation to an embodiment where the at least one sensor 112 comprises electrodes.

The TFT unit 110 may include electrodes 112 and associated sensor circuitry 114. The sensor circuitry 114 may include thin film transistors forming the desired circuitry 114.

The sensor circuitry 114 may include a modulator 114a for modulating an analog or digital signal for generating a signal carrying sensing results that may be transmitted to the electronic unit 120. The TFT unit 110 may further include circuitry 114b for extracting power from a signal from the electronic unit 120. The circuitry 114b may for instance comprise a rectifier and regulator circuit.

The electrodes 112 and associated sensor circuitry 114 may sense a biopotential of a human being. The electrodes and associated circuitry may be configured such that they sense a biopotential of a human being when the electrodes 112 are in contact with skin of a human being.

The TFT unit 110 may include a processor for pre-processing sensing results received from electrodes 112. The processor may for example also convert a signal carrying sensing results into a digital signal. The processor may for example also perform filtering of a signal carrying sensing results. The processor of the TFT unit 110 may be a specific piece of circuitry configured for a specific process.

According to an embodiment, the TFT unit 110 may comprise an electrode array comprising a plurality of electrodes 112. The associated sensor circuitry 114 may further comprise a multiplexer, to which the electrodes 112 are connected. The lines for connecting each of the electrodes to the multiplexer may be formed in thin film transistor electronics of the TFT unit 110. The multiplexer may be configured to output a signal to the modulator 114a for generating a signal carrying sensing results that may be transmitted to the electronic unit 120. The multiplexer may thus selectively provide output based on signals detected by electrodes 112 in the array.

The TFT unit 110 and the electronic unit 120 may each include elements for forming a wireless coupling between the TFT unit 110 and the electronic unit 120. The wireless coupling between the TFT unit 110 and the electronic unit 120 may be an inductive coupling or a capacitive coupling. As illustrated in Fig. 3, the TFT unit 110 may thus comprise an antenna 118, here illustrated in the form of an inductor 118, and the electronic unit may comprise an antenna 126, here illustrated in the form of a corresponding inductor 126. The inductors 118, 126 may be arranged in the TFT unit 110 and the electronic unit 120, respectively, such that the inductors 118, 126 may be arranged in close proximity to each other when the electronic unit 120 is physically connected to the TFT unit 110. The TFT unit 110 and the electronic unit 120 may communicate sensing data through the wireless coupling. The TFT unit 110 may be powered by the electronic unit 120 through the wireless coupling.

The TFT unit 110 and the electronic unit 120 may communicate using radio frequency signals that are transferred between the inductors 118, 126 of the TFT unit 110 and the electronic unit 120, respectively. The communication may be implemented, e.g. through radio frequency identification (RFID) or through near field communication (NFC).

The electronic unit 120 may include a battery 124. The battery 124 may be chargeable wirelessly or through a physical connection. The battery 124 may provide power to the electronic unit 120 and may also be used for powering the TFT unit 120 through the wireless coupling.

The electronic unit 120 may include a receiver 128 which may demodulate a received signal. The receiver 128 may thus demodulate a signal received from the TFT unit 110 so as to extract sensing results communicated from the TFT unit 110.

The electronic unit 120 may include a processor 130 for processing sensing results. The processor 130 of the electronic unit 120 may be a general-purpose processing unit, such as a central processing unit (CPU), which may be loaded with a computer program product in order to allow the processor 130 to perform the desired operations. The processor 130 may alternatively be a special-purpose circuitry for providing only specific logical operations. Thus, the processor 130 may e.g. be provided in the form of an application-specific integrated circuit (ASIC), an application-specific instruction-set processor (ASIP), a field-programmable gate array (FPGA), or a digital signal processor (DSP).

The electronic unit 120 may include a communication unit 132 which may communicate sensing results to an external unit 200, e.g. through wired communication or through a wireless communication such as radio communication.

The electronic unit 120 may include a memory 134 for storing sensing results. Thus, sensing results may be stored in the electronic unit 120 such that sensing results need not continuously be transmitted to an external unit 200. The memory 134 may be used to store all sensing results that are gathered during a session of using the system 100. The memory 134 may alternatively be used as a buffer memory 134 for temporarily storing sensing results so as to ensure that sensing results are not lost if a connection to the external unit 200 is temporarily lost.

The electronic unit 120 may include circuitry 136 for generating a signal for powering the TFT unit 110 through the wireless coupling. The circuitry 136 may generate a radio frequency signal and may comprise a power amplifier for amplifying the signal.

The system may function as such that the TFT unit 110 senses a biopotential of a human being. The TFT unit 110 may then communicate a signal carrying a sensing result to the electronic unit 120. The TFT unit 110 may pre-process the sensing result before communication. The electronic unit 120 may receive the signal. The electronic unit 120 may perform processing of the sensing results. The electronic unit 120 may store the sensing results, unprocessed or not, in a memory. The electronic unit 120 may also communicate the sensing results, unprocessed or not, to an external unit 200. The electronic unit 120 may store the sensing results prior to communication with an external unit 200 or it may communicate the sensing results without storing.

The external unit 200 may be used for storing sensing results. The external unit 200 may further be used for processing the sensing results, e.g. for extracting information relevant to be presented. The external unit 200 may also be used for further transmitting sensing results to other units, e.g. communicating sensing results over a computer network.

The external unit 200 may further be used for powering of the electronic unit 120. The external unit 200 may thus be connected to the electronic unit 120 or arranged in close proximity to the electronic unit 120 for recharging the battery 124, e.g. between sessions of gathering sensing results.

It should also be realized that the external unit 200 may be used for any combination of these functions.

In more detail the system 100 may function as such that the electrodes 112 of the TFT unit 110 senses a biopotential of a human being.

The TFT unit 110 may then condition an analog signal from the electrodes 112. The TFT unit 110 may modulate signal from the electrodes 112 to form a modulated signal carrying the sensing results and to make the signal suitable for transmission over the wireless coupling. The TFT unit 110 may convert the analog signal into a digital signal before modulating the signal. The TFT unit 110 may pre-process the signal to extract useful information and leave out unwanted information.

The TFT unit 110 may then communicate the signal carrying the sensing results to the electronic unit 120 through the wireless coupling between them.

The electronic unit 120 may, after receiving the signal over the wireless coupling, process the received signal. For example, the signal may be analyzed and interesting data may be extracted from the signal, leaving out unwanted data. The extracted data may be compressed by the electronic unit 120 for easier transfer and/or storage. The electronic unit 120 may temporarily store the processed data in the memory 134.

After receiving the signal from the TFT unit 110, the electronic unit 120 may communicate sensing results to an external unit 200. The communication of the sensing results may or may not be performed after processing of the received signal by the electronic unit 120. The communication of the signal from the electronic unit 120 to the external unit 200 may be performed wirelessly, for example using a radio communication. However, the communication may also or alternatively be achieved through a wired contact.

After receiving the signal from the TFT unit 110, the electronic unit 120 may store the sensing results in the memory 134. The storage may be done after processing of the sensing results. The stored sensing results may be communicated to the external unit 200, wherein the communication may be performed at any chosen point of time.

According to an embodiment, the TFT unit 110 may comprise one or more photo-detectors instead of the electrodes. The one or more photo-detectors may be configured to detect an intensity of light incident on the photo-detector. The photo-detector may thus be configured e.g. to detect absorbance or reflectance of light relating the detected light to light intensity from a light source illuminating the body part to which the TFT unit 110 is attached. The detected light from a plurality of photo-detectors may be compared, which may be used for e.g. determining an amount of a substance in the body part based on relation of detected amounts of light for different wavelengths.

The associated sensor circuitry 114 may control an exposure period for the one or more photo-detectors and control when a signal from the one or more photo-detectors is to be output. The sensor circuitry 114 may further include the modulator 114a for modulating analog or digital signals from the one or more photo-detectors for generating a signal carrying sensing results that may be transmitted to the electronic unit 120.

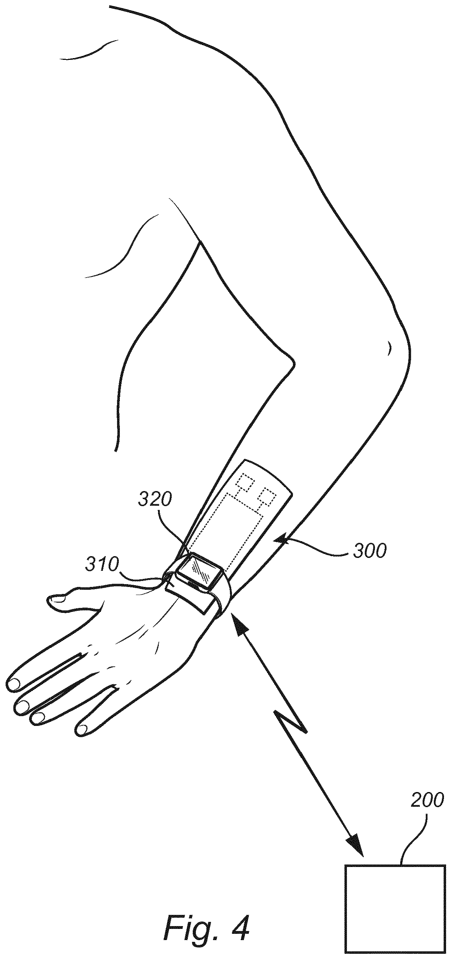

Fig. 4 illustrates a system 300 according to a second embodiment for sensing a parameter of a living being. The system 300 includes a thin film transistor (TFT) unit 310 and an electronic unit 320. An external unit 200 is also shown in Fig. 4 and may or may not, similar to the external unit of the first embodiment, be part of the system 300.

The system 300 of the second embodiment is similar to the system 100 of the first embodiment and, in the following, only differences between the systems will be described. It should be realized that, apart from the below described differences, any features of the system 100 of the first embodiment may also apply or be used in the system 300 of the second embodiment.

The TFT unit 310 and the electronic unit 320 of the second embodiment are not configured for being physically connected. Instead, the TFT unit 310 is configured for being attached to a body part of a living being, whereas the electronic unit 320 is configured for fixed arrangement on the body part. Thus, when the TFT unit 310 is attached to the living being, the electronic unit 320 may be arranged on the same body part to which the TFT unit 310 is attached, such that the TFT unit 310 and the electronic unit 320 are arranged in close relation to each other.

The TFT unit 310 and the electronic unit 320 may thus be arranged such that the electronic unit 320 wholly or partially overlaps the TFT unit 310 on the body part. The arrangement of the TFT unit 310 and the electronic unit 320 may then imply that a wireless contact between the TFT unit 310 and the electronic unit 320 may be formed by means of the antennas of the TFT unit 310 and the electronic unit 320 being arranged in close relation to each other.

The TFT unit 310 of the second embodiment may be similar to the TFT unit 110 of the first embodiment, except the TFT unit 310 of the second embodiment need not include any physical connector interface for forming a connection to the electronic unit 320.

The electronic unit 320 of the second embodiment may be similar to the electronic unit 120 of the first embodiment, except the electronic unit 320 of the second embodiment need not include any physical connector interface for forming a connection to the TFT unit 310. Instead, the electronic unit 320 may be configured to be worn by the living being, such that the electronic unit 320 may be configured for fixed arrangement on the body part to which the TFT unit 310 may be attached.

The configuration of the TFT unit 310 and the electronic unit 320 may depend on the body part on which the TFT unit 310 and the electronic unit 320 are to be arranged.

For instance, the TFT unit 310 may be formed as a patch to be attached on a wrist or arm of a human being and the electronic unit 320 may be formed as a wristwatch or bracelet for being worn on the arm, as illustrated in Fig. 4.

Alternatively, the TFT unit 310 may be formed as a patch to be attached to a chest of the human being and the electronic unit 320 may be formed as a band to be worn over the chest of the human being.

According to a further alternative, the TFT unit 310 may be formed as a patch to be attached to a forehead of the human being and the electronic unit 320 may be formed as goggles or glasses to be worn on a head of the human being.

In any of these embodiments, the electronic unit 320 may be implemented by adaptation of presently available wearable devices. For instance, the electronic unit 320 may be implemented in a smart watch or smart bracelet; in a smart chest band (as used e.g. with pulse measuring equipment), or in virtual reality goggles.

The electronic unit 320 may be specifically provided with an antenna for forming the wireless contact to the TFT unit 310. The antenna may be arranged in the electronic unit 320 such that it will be in close relation to the TFT unit 310, when the electronic unit 320 is arranged on the body part.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, the TFT unit 110 may comprise a plurality of different sensors, such as comprising both electrodes and one or more photo-detectors.

## Claims

1. A system (100; 300) for sensing of a parameter of a living being, said system comprising:
a thin film transistor, TFT, unit (110; 310), said TFT unit (110; 310) being configured for attachment to a body part of the living being and said TFT unit (110; 310) comprising at least one sensor (112) with associated sensor circuitry (114) for sensing the parameter of the living being, wherein the TFT unit (110; 310) comprises an antenna (118) for short-range communication; and
an electronic unit (120; 320), wherein the electronic unit (120) and the TFT unit (110) are configured for detachably physically connecting the electronic unit (120) to the TFT unit (110) or the electronic unit (320) is configured for fixed arrangement on the body part of the living being in physical contact with the TFT unit (310), wherein the electronic unit (120; 320) comprises an antenna (126) for short-range communication,
wherein the TFT unit (110; 310) and the electronic unit (120; 320) form a wireless contact between the antenna (118) of the TFT unit (110; 310) and the antenna (126) of the electronic unit (120; 320) for powering the TFT unit (110; 310) by the electronic unit (120; 320) and communicating sensing results from the TFT unit (110; 310) to the electronic unit (120; 320).

2. The system according to claim 1, wherein the TFT unit (110; 310) is formed on a flexible substrate and the sensor circuitry (114) is formed of thin film transistors.

3. The system according to any one of the preceding claims, wherein the TFT unit (110; 310) is disposable and the electronic unit (120; 320) is re-usable with different TFT units (110; 310).

4. The system according to any one of the preceding claims, wherein the TFT unit (110; 310) is configured to form a waterproof enclosure of the sensor circuitry (114).

5. The system according to any one of the preceding claims, wherein the sensor circuitry (114) is configured to perform modulation of an analog or digital signal for communicating sensing results to the electronic unit (120; 320).

6. The system according to any one of the preceding claims, wherein the electronic unit (120; 320) comprises a processor unit (130) for processing received sensing results.

7. The system according to any one of the preceding claims, wherein the electronic unit (120; 320) comprises a memory (134) for storing sensing results.

8. The system according to any one of the preceding claims, wherein the electronic unit (120; 320) comprises a communication unit (132) for communication with an external unit (200).

9. The system according to any one of the preceding claims, wherein the electronic unit (120; 320) comprises a battery (124).

10. The system according to claim 9, wherein the battery (124) is rechargeable and the electronic unit (120; 320) is configured for wireless charging of the battery (124).

11. The system according to any one of the preceding claims, wherein the electronic unit (120; 320) comprises a housing (122) providing a waterproof enclosure of components of the electronic unit (120; 320).

12. The system according to any one of the preceding claims, wherein the wireless contact between the TFT unit (110; 310) and the electronic unit (120; 320) is formed through an inductive or capacitive coupling between the antenna (118) of the TFT unit (110; 310) and the antenna (126) of the electronic unit (120; 320).

13. The system according to any one of the preceding claims, wherein communication between the TFT unit (110; 310) and the electronic unit (120; 320) is performed over radio frequency identification, RFID, communication or near field communication, NFC.

14. The system according to any one of the preceding claims, wherein the electronic unit (120) and the TFT unit (110) are configured for detachably physically connecting the electronic unit (120) to the TFT unit (110) by using at least one of: an adhesive, a magnetic coupling, and one or more interlocking elements, such as hook and loop fasteners.

15. An electronic unit for use in a system for sensing of a parameter of a living being, said electronic unit (120) comprising:
a physical connection interface configured for detachably physically connecting the electronic unit to a thin film transistor, TFT, unit (110); and
a wireless communication unit for forming an inductive or capacitive coupling to the TFT unit (110) for powering the TFT unit (110) and receiving sensing results from the TFT unit (110).
